# FASCICULE DE BREVET EUROPEEN

(11) **EP 2 827 836 B1**
(45) Date de publication et mention de la délivrance du brevet: **02.01.2019**
(21) Numéro de dépôt: 13723204.7
(22) Date de dépôt: 08.03.2013
(51) Int. Cl.: A61K 8/64, A61Q 19/08, A61Q 17/04, A61Q 19/00

(54) **COMPOSITION COSMÉTIQUE COMPRENANT UN SYSTÈME ACTIVATEUR DE LA PROTÉINE TRF2 A ACTION SYNERGIQUE CONSTITUE D'UNE ASSOCIATION D'UN EXTRAIT PEPTIDIQUE DE SOJA ET DE LEVURE ET SES UTILISATIONS**
KOSMETISCHE ZUSAMMENSETZUNG MIT EINEM SYNERGISTISCHEN TRF2-PROTEINAKTIVIERUNGSSYSTEM AUS EINER KOMBINATION AUS EINER PEPTIDISCHEN SOJABOHNE UND HEFEEXTRAKT SOWIE VERWENDUNGEN DAVON
COSMETIC COMPOSITION COMPRISING A SYNERGISTIC TRF2 PROTEIN ACTIVATION SYSTEM CONSISTING OF A COMBINATION OF A PEPTIDIC SOYBEAN AND YEAST EXTRACT AND THE USES THEREOF

(30) Priorité: 19.03.2012 FR 1200814
(43) Date de publication de la demande: 28.01.2015
(73) Titulaire: ISP Investments LLC, Wilmington, DE 19805 (US)
(72) Inventeur: BOTTO, Jean-Marie, F-06560 Valbonne (FR); DOMLOGE, Nouha, F-06650 OPIO (FR); PORTOLAN, Frédérique, F-06560 Valbonne (FR)
(74) Mandataire: Miquel, Corinne
(86) Numéro de dépôt international: PCT/IB2013/000756
(87) Numéro de publication internationale: WO 2013/140252

(56) Documents cités:
- FR-A1- 2 887 772
- FR-A1- 2 915 384
- FR-A1- 2 927 254
- FR-A1- 2 944 795
- DATABASE GNPD [Online] MINTEL; décembre 2006 (2006-12), "Complete Anti-Ageing Eye Contour Care", XP002689400, Database accession no. 632286
- DATABASE GNPD [Online] MINTEL; août 2010 (2010-08), "Ultra All Night Repair and Moisture Cream for Face and Throat", XP002689401, Database accession no. 1390859

## Description

La présente invention se situe dans le domaine de la cosmétique. Elle se rapporte à une composition cosmétique comprenant un système activateur de la protéine TRF2 à action synergique constitué par l'association d'un extrait peptidique de soja et de levure, plus particulièrement destinée à prévenir et/ou traiter les dommages subis par l'ADN des cellules cutanées et les signes cutanés liés au vieillissement.

La sénescence est un processus biologique qui atteint toutes les cellules « normalement » constituées d'un être humain. Celle-ci se traduit d'un point de vue physique par un vieillissement global du corps humain, que ce soit les cellules de la peau, des cheveux, des organes...

Il a été démontré scientifiquement que la sénescence est causée notamment par le raccourcissement des télomères (sénescence réplicative) ou par l'exposition aigüe ou chronique à des signaux de stress physiologiques comme par exemple les stress oxydatifs. Un des moyens explorés afin de limiter la sénescence des cellules et par conséquent limiter les manifestations du vieillissement chez l'être humain, consiste à agir sur le raccourcissement des télomères et/ou limiter l'action des stress oxydatifs sur l'ADN.

Les télomères sont formés de répétitions de la séquence TTAGGG et de protéines spécifiques. Les télomères sont situés aux extrémités des chromosomes et assurent la stabilité de ceux-ci. Parmi les protéines qui s'associent aux télomères, on trouve les protéines TRF1 et TRF2 (TRF pour telomere-binding factor) qui se fixent directement sur l'ADN télomérique double brin, ou encore la protéine POT1 qui elle, se lie, à l'extrémité 3' simple brin. Au cours des divisions cellulaires, des motifs TTAGGG sont perdus en cours de route lors de la réplication. Cette perte de télomères est partiellement compensée par une transcriptase inverse, la télomérase, qui synthétise *de novo* des répétitions télomériques sur des extrémités de télomères préexistants, assurant ainsi une longueur optimale.

Le problème qui se pose, est que l'activité de cette télomérase est extrêmement faible dans les cellules somatiques, ceci conduisant à un raccourcissement télomérique à chaque réplication chromosomique.

Une des stratégies utilisée à l'heure actuelle pour limiter le vieillissement de la peau ainsi que ses manifestations sur celle-ci, consiste à augmenter l'activité télomérase des cellules de la peau. Cependant, on s'expose ainsi à une immortalisation desdites cellules, ce qui n'est pas souhaitable car les cellules immortalisées peuvent s'apparenter à des cellules cancéreuses.

La Demanderesse a exploré une autre piste pour tenter de retarder la sénescence cellulaire, en modulant la quantité de protéines associées aux télomères, et plus particulièrement la protéine TRF2. Ainsi, la Demanderesse a mis au point un système activateur de la protéine TRF2 à action synergique constitué par l'association d'un extrait peptidique de soja et de levure permettant d'augmenter la quantité des protéines associées aux télomères dans les cellules cutanées.

D'autres composés modulateurs des protéines TRFs ou encore stabilisateurs des télomères ont déjà été proposés auparavant comme exposés dans les demandes de brevet US20020076719, WO9836066, ou encore WO2004092395. De plus, l'utilisation cosmétique d'extraits de soja ou de levure a été décrite comme ayant une action anti-âge sur la peau (demandes de brevet FR2915378, FR2915384, FR2915381, FR2887775, FR2887772, FR2826576, FR2944795, FR2927254) et des compositions cosmétiques comprenant un extrait aqueux de soja, de malt et de levure ont également été décrites pour augmenter l'élasticité de la peau (KR2003045437). Des compositions anti-âges comprenant, entre autres, un hydrolysat de protéines de soja et un hydrolysat de protéines de levure ont également été décrits (Jeanne Piaubert « Complete anti-Ageing Eye Contour Care » et Elizabeth Arden « Ultra All Night Repair and Moisture Cream For Face and Throat »).

Cependant, aucun des documents cités précédemment ne divulgue ni ne suggère un système activateur de la protéine TRF2 à action synergique constitué par l'association d'un extrait peptidique de soja et de levure à des concentrations déterminées, tel qu'exposé dans la présente demande de brevet, ni l'utilisation d'une composition cosmétique comprenant un tel système activateur de la protéine TRF2 pour prévenir et/ou traiter les dommages subis par l'ADN des cellules cutanées et les signes cutanés liés au vieillissement. Le système activateur de la protéine TRF2 utilisé selon l'invention offre notamment les avantages suivants :
- il augmente la quantité de protéines associées aux télomères (TRFs) et en particulier la protéine TRF2 dans les cellules cutanées ;
- il limite l'augmentation et l'agrégation de la protéine du cytosquelette Vimentine dans les cellules ayant un phénotype de cellules âgées ;
- il limite l'apparition de dommages dans des biopsies de peaux soumises à du méthylglyoxal ;
- il limite la dégradation des télomères sans modifier la quantité et/ou l'activité de la télomérase ; et
- il permet en conséquence de prévenir et/ou traiter les dommages subis par l'ADN des cellules cutanées et les signes cutanés liés au vieillissement.

La présente invention a pour premier objet une composition cosmétique comprenant, dans un milieu physiologiquement acceptable, un système activateur de la protéine TRF2 à action synergique constitué par l'association d'un extrait peptidique de soja et de levure obtenu par l'hydrolyse concomitante de 90% de graine de soja (*Glycine Max L*.) en poids du poids total de matières premières et de 10% de levure du genre Saccharomyces en poids du poids total de matières premières, caractérisée en ce que l'extrait peptidique de soja et de levure comprend de 0,5 à 5,5 g/L de composés peptidiques dont le poids moléculaire est inférieur à 5 kDa.

L'invention a pour deuxième objet l'utilisation cosmétique non-thérapeutique d'une composition selon l'invention, pour prévenir et/ou réparer la survenue des cassures doubles brins au niveau de l'ADN des cellules cutanées. Enfin, l'invention a pour troisième objet des méthodes de soin cosmétique non-thérapeutique pour prévenir et/ou réparer les dommages subis par l'ADN des cellules cutanées liés au vieillissement comprenant l'application topique sur au moins une partie de la peau du visage ou du corps de la composition selon l'invention dans laquelle la composition est appliquée le matin en tant que soin anti-âge de jour et/ou le soir au coucher en tant que soin réparateur de nuit ou appliquée avant une exposition au soleil, en tant que soin avant-soleil et/ou après une exposition au soleil, en tant que soin après-soleil.

L'invention et les avantages qui en découlent seront mieux compris à la lecture de la description et des modes de réalisation non limitatifs qui suivent.

La présente invention concerne donc une composition cosmétique comprenant, dans un milieu physiologiquement acceptable, un système activateur de la protéine TRF2 à action synergique constitué par l'association d'un extrait peptidique obtenu par hydrolyse de différentes matières premières sélectionnées, à savoir le soja et les levures.

L'extrait peptidique de soja selon l'invention provient de l'hydrolyse de graines de soja (*Glycine Max L.)* et, d'autre part, l'extrait peptidique de levure selon l'invention provient de l'hydrolyse de biomasse de levures du genre Saccaromyces, et plus particulièrement de l'espèce *Saccharomyces cerevisiae.*

De préférence, le soja n'est pas soumis à une fermentation préalable et les levures sont lyophilisées.

L'hydrolyse du soja et de la levure a lieu concomitamment, par exemple selon le procédé de l'exemple 1.

Le système activateur de la protéine TRF2 selon l'invention est constitué par l'hydrolyse de 90% de soja en poids du poids total de matières premières et de 10% de levure en poids du poids total de matières premières.

L'extrait peptidique de soja et de levure qui constitue le système activateur de la protéine TRF2 obtenu selon l'invention est analysé qualitativement et quantitativement pour ses caractéristiques physico-chimiques et sa teneur en composés peptidiques ; les peptides, acides aminés et fragments de protéines sont dosés selon les techniques classiques, bien connues de l'homme du métier.

L'extrait peptidique de soja et de levure préféré selon l'invention comprend essentiellement des composés peptidiques de bas poids moléculaires, c'est-à-dire ne comprend avantageusement que des peptides de bas poids moléculaire. Préférentiellement, il comprend essentiellement des composés peptidiques de poids moléculaire inférieur à 5 kDa, c'est-à-dire avantageusement l'extrait peptidique de soja et de levure selon l'invention ne comprend que des composés peptidiques dont le poids moléculaire est inférieur à 5 kDa.

L'extrait peptidique de soja et de levure selon l'invention est dilué pour contenir entre 0,5 et 5,5 g/L de composés peptidiques en poids d'extrait sec.

La composition cosmétique selon l'invention comprenant le système activateur de la protéine TRF2 et un milieu physiologiquement acceptable se présente sous une forme adaptée à l'application par voie topique sur au moins une partie de la peau du visage ou du corps.

Un milieu physiologiquement acceptable selon l'invention désigne en particulier des milieux qui conviennent à une utilisation en contact avec la peau ou les phanères humains, sans risque par exemple de toxicité, d'incompatibilité, d'instabilité, ou encore de réponse allergique.

Selon un mode de réalisation avantageux de l'invention, l'extrait peptidique de soja et de levure qui constitue le système activateur de la protéine TRF2 est préalablement solubilisé dans un ou plusieurs solvants physiologiquement acceptables, classiquement utilisés par l'homme du métier, comme l'eau, le glycérol, l'éthanol, le propylène glycol, le butylène glycol, le dipropylène glycol, les diglycols éthoxylés ou propoxylés, les polyols cycliques, la vaseline, une huile végétale ou tout mélange de ces solvants.

Selon un autre mode de réalisation avantageux de l'invention, l'extrait peptidique de soja et de levure selon l'invention est solubilisé dans un vecteur cosmétique comme les liposomes, ou adsorbé sur des polymères organiques poudreux, des supports minéraux comme les talcs et bentonites, et plus généralement solubilisé dans, ou fixé sur, tout vecteur physiologiquement adapté.

De manière préférée, la composition selon l'invention destinée à être appliquée de façon topique se présente sous forme de crème, émulsion huile-dans-eau, ou eau-dans-huile ou émulsion multiple, solution, suspension, microémulsion, gel aqueux ou anhydre, sérum, ou encore de dispersion de vésicules, de patch, de spray, d'onguent, de pommade, de lotion, de colloïde, de lait, de stick ou de poudre.

Selon un mode de réalisation avantageux de l'invention, l'extrait peptidique de soja et de levure est présent dans la composition cosmétique selon l'invention à une concentration comprise entre 0,0001% à 20% environ, et préférentiellement à une concentration comprise entre 0,05% et 5% environ, encore plus préférentiellement à une concentration comprise entre 1% et 3% environ par rapport au poids total de la composition finale.

Encore plus préférentiellement, la composition cosmétique selon l'invention contient en outre, au moins un autre agent actif. On peut citer, de manière non limitative, les classes d'ingrédients suivantes : d'autres agents actifs peptidiques, des extraits de végétaux, des agents cicatrisants, anti-âge, antirides, apaisants, anti-radicalaires, anti-UV, des agents stimulant la synthèse de macromolécules dermiques ou le métabolisme énergétique, des agents hydratants, antibactériens, antifongiques, anti-inflammatoires, anesthésiques, des agents modulants la différenciation, la pigmentation ou la dépigmentation cutanée, des agents stimulants la pousse des ongles ou des cheveux etc. Préférentiellement, on utilisera un agent présentant une activité dans le domaine des antirides, tel qu'un agent anti-radicalaire ou antioxydant, ou un agent stimulant la synthèse de macromolécules dermiques, ou encore un agent stimulant le métabolisme énergétique. Plus particulièrement, l'agent actif est choisi parmi les vitamines, les phytostérols, les flavonoïdes, la DHEA et/ou un de ses précurseurs ou un de ses dérivés chimiques ou biologiques, un inhibiteur de métalloprotéinase, ou un rétinoïde.

En outre, des additifs tels que des solvants, des diluants, des colorants, des filtres solaires, des agents auto-bronzants, des pigments, des charges, des conservateurs, des absorbeurs d'odeurs, des agents épaississants, émulsifiants, humectants, émollients, des parfums, des antioxydants, des agents filmogènes, chélatants, séquestrants, conditionnants peuvent être ajoutés à la composition selon l'invention.

Dans tous les cas, l'homme du métier veillera à ce que ces adjuvants ainsi que leurs proportions soient choisis de telle manière à ne pas nuire aux propriétés avantageuses recherchées de la composition comprenant le système activateur de la protéine TRF2 à action synergique constitué d'un extrait peptidique de soja et de levure selon l'invention. Ces adjuvants peuvent, par exemple, être compris entre 0,01% et 20% du poids total de la composition. Lorsque la composition selon l'invention est une émulsion, la phase grasse peut représenter de 5% à 80% en poids et de préférence de 5% à 50% en poids par rapport au poids total de la composition. Les émulsionnants et co-émulsionnants utilisés dans la composition seront choisis parmi ceux classiquement utilisés dans le domaine considéré. Par exemple, ils peuvent être utilisés dans une proportion allant de 0,3% à 30% en poids par rapport au poids total de la composition.

La composition cosmétique selon l'invention, et plus particulièrement le système activateur de la protéine TRF2 à action synergique constitué par l'association d'un extrait peptidique de soja de levure obtenu par hydrolyse d'au moins 80% de soja en poids du poids total de matières premières et d'au plus 20% de levure en poids du poids total de matières premières, augmente la quantité de protéines TRF (TRF2 et/ou POT1) associées aux télomères, préférentiellement TRF2, dans les cellules cutanées, soit par augmentation de la synthèse protéique de celle-ci (par modulation directe ou indirecte de l'expression génique), soit par d'autres processus biologiques tels que la stabilisation des transcrits d'ARN messager.

Le système activateur de la protéine TRF2 présente une action synergique en ce que l'association d'un extrait peptidique de soja et de levure obtenu par hydrolyse d'au moins 80% de soja en poids du poids total de matières premières et d'au plus 20% de levure en poids du poids total de matières premières, permet une augmentation de l'efficacité du système activateur d'environ 20% en ce qui concerne l'augmentation de l'expression de TRF2 dans des kératinocytes et fibroblastes en culture, par rapport à un extrait peptidique de soja à 100% pour une même quantité totale en poids du poids total de matières premières.

Ainsi, un autre objet de l'invention se rapporte à l'utilisation cosmétique de la composition selon l'invention pour prévenir et/ou réparer les dommages subis par l'ADN des cellules cutanées liés au vieillissement en augmentant la quantité de TRF2 dans les cellules pour limiter la dégradation des télomères sans modifier la quantité et/ou l'activité de la télomérase, ce qui est important car il n'y aura ainsi aucun risque d'immortalisation des cellules.

Les dommages subis par l'ADN cellulaire chez l'Homme se réparent tous les jours, mais pas intégralement. Les dégâts non réparés vont alors s'accumuler au fil des ans. Il est ainsi essentiel de prévenir ces dommages et de les réparer.

Par prévenir les dommages subis par l'ADN des cellules cutanées, on désigne le rôle protecteur du système activateur de la protéine TRF2 selon l'invention au niveau des cellules de la peau pour limiter l'apparition de dommages au niveau de l'ADN.

Par réparer les dommages subis par l'ADN des cellules cutanées, on désigne le rôle réparateur du système activateur de la protéine TRF2 selon l'invention au niveau de l'endommagement préalablement subi par les cellules de la peau.

Les cellules cutanées selon l'invention désignent les cellules de l'épiderme et du derme, et en particulier les kératinocytes et les fibroblastes.

Les dommages subis par l'ADN des cellules cutanées selon l'invention désignent les dommages dus à des réactions photochimiques entre les bases de l'ADN, comme par exemple la formation de dimères pyrimidiques de cyclobutane, les cassures doubles brins au niveau de l'ADN ou encore tout autre dommage subi par l'ADN lors d'agressions extérieures que peut produire l'environnement.

A titre d'exemple, on peut citer des agressions telles que la pollution, les agressions provoquant des stress oxydatifs, les rayonnements UV, en particulier UVB, liés par exemple à une surexposition au soleil, ou encore les produits à caractère irritant tels que les tensioactifs, les conservateurs ou les parfums.

La composition cosmétique selon l'invention est utilisée préférentiellement afin de prévenir et/ou réparer la survenue des cassures doubles brins au niveau de l'ADN des cellules cutanées lors de dommages. En effet, lors de dommages infligés aux cellules de la peau, notamment les dommages dus aux rayonnements UV ou encore les stress oxydatifs, l'ADN desdites cellules cutanées est susceptible de subir des dommages se manifestant par des cassures doubles brins. Le système activateur de la protéine TRF2 à action synergique constitué par l'association d'un extrait peptidique de soja et de levure selon l'invention a démontré son action protectrice de l'ADN vis-à-vis de ce genre de dommages.

La composition cosmétique selon l'invention est également utilisée pour prévenir et/ou traiter les signes cutanés du vieillissement et les signes cutanés du photo-vieillissement induits par les rayonnements UV.

Le photo-vieillissement selon l'invention désigne le vieillissement prématuré de la peau causé par l'exposition prolongée et cumulative au soleil.

Les signes cutanés du vieillissement et du photo-vieillissement selon l'invention incluent, mais ne se limitent pas à, toutes les manifestations visibles sur la peau causées par le vieillissement. On entend, en particulier, les rides, les rides profondes, les ridules, les crevasses, le relâchement des tissus cutanés et sous-cutanés, la perte de l'élasticité cutanée et l'atonie, la perte de fermeté et de tonicité, et l'atrophie dermique.

La composition cosmétique selon l'invention vise préférentiellement à prévenir et/ou traiter les rides, les rides profondes, les ridules, les crevasses, le relâchement des tissus cutanés et sous-cutanés, la perte de l'élasticité cutanée et l'atonie, la perte de fermeté et de tonicité, et l'atrophie dermique.

Enfin, un dernier objet de la présente invention se rapporte à une méthode de soin cosmétique pour prévenir et/ou réparer les dommages subis par l'ADN des cellules cutanées liés au vieillissement comprenant l'application topique sur au moins une partie de la peau du visage ou du corps de la composition selon l'invention, dans laquelle la composition est appliquée le matin en tant que soin anti-âge de jour et/ou le soir au coucher en tant que soin réparateur de nuit. En cas d'application en soin de jour, la composition permet en particulier une protection de la peau vis-à-vis des agressions de l'environnement, notamment les rayonnements UV, en particulier UVB, en limitant l'apparition de dommages au niveau de l'ADN des cellules cutanées. En tant que soin de nuit, la composition joue plus particulièrement un rôle de réparation des éventuels dommages que la peau aura subie lors de la journée.

Dans un autre mode de réalisation avantageux de la méthode de soin cosmétique selon l'invention, la composition est appliquée avant une exposition au soleil, en tant que soin avant-soleil et/ou après une exposition au soleil, en tant que soin après-soleil, et constitue donc un excellent soin avant-soleil permettant en particulier de prévenir les dommages au niveau de l'ADN des cellules cutanées, et/ou après une exposition au soleil, en tant que soin après-soleil afin de réparer plus particulièrement les éventuels dommages subis par la peau au niveau de l'ADN.

Les exemples suivants décrivent et démontrent l'efficacité du système activateur de la protéine TRF2 à action synergique tel que décrit selon l'invention mais ne doivent pas être interprétés comme une limitation de la présente invention.

### Exemple 1 : procédé d'obtention d'un extrait peptidique de soja et de levure selon l'invention :

Afin d'obtenir le système activateur de la protéine TRF2 à action synergique selon l'invention, 90% en poids du poids total de matières premières de graines de soja préalablement moulues sont mélangées à 10% en poids du poids total de matières premières de biomasse de levures. Le mélange des matières premières est mis en solution dans de l'eau dans un ratio en poids de 1/60, soit 1 kg de mélange de matières premières dans 60 kg d'eau. Le pH de la solution est ajusté à une valeur comprise entre 7 et 7,5. Après ajustement du pH, 2% de bromélaine et 2% de POLYCLAR® 10 (polyvinylpyrrolidone - PVPP - insoluble) sont ajoutés dans le milieu réactionnel. Le milieu réactionnel est ensuite chauffé deux heures à 55°C puis désactivé pendant deux heures à 80°C. Une étape de filtration permet de récupérer le filtrat composé de 20 à 25 g/L de matière sèche, de 19 à 22 g/L de protéines et de 2 à 3 g/L de sucres.

La nature protéique de ce filtrat est mise en évidence par électrophorèse sur gel de polyacrylamide. Pour cette analyse, les gels NuPAGE® Bis-Tris Pre-cast (Invitrogen) sont utilisés. L'extrait peptidique intermédiaire de soja et de levure est chauffé à 70°C pendant 10 minutes dans des conditions réductrices dénaturantes dans un tampon de préparation d'échantillon NuPAGE® LDS. Une solution de NuPAGE® Antioxydant est ajoutée dans la cuve intérieure afin d'éviter que les protéines réduites ne se ré-oxydent durant l'électrophorèse. La migration des composés peptidiques est réalisée à l'aide du tampon de migration NuPAGE® MES avec le standard SeeBlue Plus2 comme marqueur de poids moléculaire. La coloration des composés peptidiques est effectuée à l'aide de Bleu de Coomassie® R 250. Le profil peptidique ainsi obtenu met en évidence une répartition des poids moléculaires inférieurs à 6 kDa.

L'extrait peptidique intermédiaire de soja et de levure précédemment obtenu est alors purifié par des filtrations successives à l'aide de filtres-plaques Seitz-Orion de porosité décroissante (jusqu'à 0,2 µm) afin d'obtenir une solution brillante et limpide. A cette étape, l'extrait de soja et de levure est caractérisé par un poids sec de 20-22 g/kg, un taux de composés peptidiques de 18-20 g/L et un taux de sucres de 1-2 g/L.

Cette solution est alors purifiée en éliminant les composés peptidiques de poids moléculaire supérieur à 5 kDa à l'aide de filtration à flux tangentiel. Pour cela, la solution de soja et de levure est pompée sous pression à travers un support Pellicon® équipée de cassette Pellicon® 2 Biomax 10 kDa. Ce premier filtrat est récupéré pour être ensuite filtré à travers une autre cassette Pellicon® 2 Biomax 5 kDa.

En fin de purification, on obtient un extrait végétal peptidique de soja et de levure jaune pâle, brillant et limpide. Il est caractérisé par un poids sec de 18-20 g/kg, une teneur en composés peptidiques de 16-18 g/L et un taux de sucres entre 0,3 et 0,5 g/L.

On procède ensuite à une étape de filtration stérilisante pour obtenir l'extrait peptidique de soja et de levure final selon l'invention, dilué à 2,35 g/L de composés peptidiques dans 30% de glycérol.

### Exemple 2: étude de l'effet de l'extrait peptidique de soja et de levure selon l'exemple 1 sur des fibroblastes vieillis par sénescence réplicative

Des fibroblastes humains sont mis en culture dans un milieu spécifique et maintenus en culture en traitement long (pendant plus de 17 passages), à l'aide d'une application quotidienne de l'extrait peptidique de soja et de levure selon l'exemple 1 à une concentration de 1 % ou de 3 %. Une expérience de détection par immunofluorescence de la vimentine est effectuée sur les cellules aux passages de culture 6 et 17. Les cellules sont ensuite lavées avec du PBS, fixées au formaldéhyde à 3,7 % pendant 10 minutes, perméabilisées à l'aide de 0,2 % de Triton X-100 pendant 10 minutes (Fisher Chemical) et incubées avec 1 % de BSA (Euromedex) pendant 15 minutes. Un anticorps anti-vimentine (Tebu Santa Cruz) est par la suite ajouté et incubé à une dilution de 1/200^{ième} pendant 2 h à température ambiante. Après lavage au PBS, un anticorps anti-souris Donkey IgG, conjugué à un marqueur Alexa Fluor® 488 sont ajoutés à une dilution de 1/1000^{ième} et laissés en incubation pendant une heure à température ambiante. Enfin, les coupes sont montés au Fluoromount G (Electron Microscopy Science) et examinées à l'aide d'un microscope (Nikon Eclipse 80i, grossissement 40x).

### Résultats/ Conclusions :

On constate que les fibroblastes vieillis traités avec l'extrait peptidique de soja et de levure selon l'exemple 1 expriment moins de vimentine que les fibroblastes vieillis non traités. Or, l'augmentation et l'agrégation de la vimentine est associée aux modifications du cytosquelette qui accompagnent le phénomène de vieillissement. On peut donc conclure que le traitement avec l'extrait peptidique de soja et de levure selon l'invention a permis de limiter l'augmentation et l'agrégation de la vimentine dus au vieillissement des fibroblastes.

### Exemple 3 : étude de l'effet de l'extrait peptidique de soja et de levure selon l'exemple 1 sur un modèle de biopsies rendues sénescentes in vitro à l'aide de méthyglyoxal

Des biopsies de peaux humaines sont rendues sénescentes artificiellement *in vitro,* grâce à un traitement à l'aide de méthylglyoxal (MGO). Pour cela, des punch de 6 mm de biopsies de peaux humaines sont mises à incuber à l'interface air-liquide dans un milieu de culture spécifique. Elles sont ensuite traitées avec 5 mM ou 10 mM de MGO (Sigma) déposés à la surface des biopsies et dans le milieu de culture. Les biopsies sont par la suite traitées avec soit :
- condition 1 : 20 µL de PBS 1X, soit
- condition 2: 20 µL de l'extrait peptidique de soja et de levure selon l'exemple 1 à 1 %, soit
- condition 3 : 20 µL de l'extrait peptidique de soja et de levure selon l'exemple 1 à 3%.

Les biopsies sont fixées et incluses dans de la paraffine, et sont ensuite coupées en sections de 4 µm à l'aide d'un microtome. Un immunomarquage hématoxyline/éosine (H&E) est réalisé sur les sections découpées précédemment et leur morphologie et structure sont étudiées par observation microscopique (à l'aide d'un microscope Nikon Eclipse E600, objectif 40x).

### Résultats/Conclusions :

Dans la condition contrôle 1, c'est-à-dire sans ajout d'extrait peptidique selon l'invention, on constate que le MGO a provoqué des dommages importants au niveau des biopsies de peaux, plus exactement au niveau de la structure de celles-ci. Les dommages provoqués sont dose-dépendant puisqu'on observe plus de dommages avec la quantité de 10 mM de MGO.

Lorsque les biopsies sont traitées avec l'extrait peptidique de soja et de levure selon l'exemple 1, on constate que les dommages infligés aux structures desdites biopsies sont beaucoup moins importants que dans les conditions contrôle. On constate que l'effet protecteur de l'extrait peptidique de soja et de levure selon l'exemple 1 est dose-dépendant puisqu'on observe encore moins de dommages avec la dose de 3 % qu'avec la dose de 1 %.

On peut ainsi conclure que l'extrait peptidique de soja et de levure selon l'invention a eu un effet protecteur des structures cellulaires lorsque celles-ci sont soumises à des stress conduisant à des dommages importants et une sénescence précoce.

### Exemple 4 : étude de l'expression de la protéine TRF2 par siRNA dans des fibroblastes humains traités avec l'extrait peptidique de soja et de levure selon l'exemple 1

Afin de quantifier l'efficacité de l'extrait peptidique selon l'invention, sur la surexpression de TRF2 dans une population de fibroblastes humains, le gène codant pour TRF2 a été « éteint » par utilisation de siRNA (RNA silencer).

### Protocole :

Des cellules fibroblastes sont mises en culture en plaque 6 puits jusqu'à une confluence de 60 %. Le milieu de culture est renouvelé avec ajout de l'extrait peptidique de soja et de levure selon l'exemple 1 à 1 % dans les conditions décrites ci-dessous. Ensuite, 100 µL d'un mélange préalablement réalisé contenant le siRNA de TRF2 à 10 nM final et l'agent transfectant sont rajoutés délicatement au goutte à goutte, puit par puit. La plaque de culture cellulaire est incubée à 37°C et à 5 % en CO₂ pendant 72 h. Le milieu de culture est renouvelé tous les 2 jours. Quatre conditions ont été mises en place :
- condition 1 : contrôle sans siRNA et sans actif
- condition 2 : cellules transfectées avec le siRNA, sans actif
- condition 3 : cellules non transfectées mais traitées avec l'actif
- condition 4 : cellules transfectées avec le siRNA et traitées avec l'actif

La quantification de l'expression de TRF2 est observée par la technique classique d'immunotransfert (Western Blot) réalisée à l'aide d'un anticorps anti-TRF2 et selon un protocole classique. Afin d'analyser la compensation apportée par le peptide dans les fibroblastes ayant été transfectés avec le siRNA, la comparaison sera faite par rapport à des fibroblastes non traités et dont le gène n'a pas été éteint par siRNA.

### Résultats/Conclusions :

Entre les conditions 1 et 3, on constate que l'ajout de l'extrait peptidique de soja et de levure selon l'exemple 1 a entraîné une augmentation de l'expression de la protéine TRF2 de 17 % par rapport au contrôle. Entre les conditions 1 et 2, on constate bien l'effet du siRNA sur l'expression de la protéine TRF2 : en effet, celle-ci est en baisse de 26 %. Par contre, l'ajout de l'actif aux cellules transfectées avec le siRNA permet de restaurer l'expression de TRF2, et la baisse due à la présence de siRNA n'est plus que de 18 % par rapport à la condition contrôle.

En conclusion, l'extrait peptidique de soja et de levure selon l'invention a permis de compenser la diminution de l'expression de la protéine TRF2 (diminution induite par le siRNA spécifique) dans les fibroblastes traités.

### Exemple 5 : étude de l'expression de la protéine TRF2 par l'utilisation d'un siRNA dans des kératinocvtes humains traités avec l'extrait peptidique de soja et de levure selon l'exemple 1

Afin de quantifier l'efficacité de l'extrait peptidique selon l'invention, sur la modulation du taux d'expression de TRF2 dans une population de kératinocytes, le gène codant pour TRF2 a été « éteint » par utilisation de siRNA.

### Protocole :

Des kératinocytes humains en culture sont traités ou non avec un siRNA spécifique de TRF2 (siRNA custon, Qiagen) à une concentration finale de 25 nM en utilisant la technique de transfection par la Lipofectamine™ RNAiMAX (Invitrogen, Ref : 13778-075) et traités ou non avec l'ingrédient à une concentration finale de 1%, pendant 48 heures.
Quatre conditions ont été mises en place :
- condition 1 : contrôle sans siRNA et sans actif
- condition 2 : cellules transfectées avec le siRNA, sans actif
- condition 3 : cellules non transfectées mais traitées avec l'actif
- condition 4: cellules transfectées avec le siRNA et traitées avec l'actif

Les cellules sont ensuite lavées, fixées au formaldéhyde à 3.7% pendant 10 minutes à température ambiante. Une perméabilisation des noyaux est réalisée à l'aide d'une incubation avec du Triton X-100 à 0,2% pendant 10 minutes à température ambiante. Les sites non spécifiques sont bloqués avec de la BSA à 1% appliquée pendant 30 minutes. Les cellules sont incubées en présence d'un anticorps monoclonal de souris spécifique de TRF2 (Abcam, réf : ab13579), puis d'un anticorps secondaire anti-souris couplé à un fluorochrome (Invitrogen, réf : A21202). Les cellules sont alors examinées au microscope à Epi-fluorescence (Nikon Eclipse E600 microscope).

Trois images par condition sont analysées et quantifiées grâce au logiciel Image-Pro Analyzer 6.3 software (MediaCybernetics, Inc.). Des analyses statistiques sont ensuite menées sur ces données en utilisant le test t de Student.

### Résultats/Conclusions :

Entre les conditions 1 et 3, on constate que l'ajout de l'extrait peptidique de soja et de levure selon l'exemple 1 a entrainé une augmentation de l'expression de TRF2 de 34,4% par rapport au contrôle. Entre les conditions 1 et 2, on constate bien l'effet du siRNA TRF2 sur le taux d'expression de la protéine TRF2 : en effet, celle-ci est en baisse de 32,5%. Par contre, l'ajout de l'actif aux cellules transfectées avec le siRNA permet de restaurer l'expression de TRF2, et on observe une augmentation de 75,5% par rapport aux cellules transfectées et non traitées.

En conclusion, l'extrait peptidique de soja et de levure selon l'invention a permis de compenser la diminution de l'expression de la protéine TRF2 (diminution induite par le siRNA spécifique) dans les kératinocytes traités.

### Exemple 6 : étude de l'expression de la protéine TRF2 dans des fibroblastes humains traités avec l'extrait peptidique de soja et de levure selon l'exemple 1 et vieillis par induction artificielle de la sénescence

Afin de quantifier l'efficacité de l'extrait peptidique selon l'invention, sur la modulation du taux d'expression de TRF2, une induction artificielle de la sénescence d'une population de fibroblastes en traitant les cellules avec un siRNA spécifique du gène FOXO3a pendant 48h a été réalisée.

### Protocole :

Des fibroblastes humains en culture sont traités ou non avec un siRNA spécifique de FOXO3a (HSS177176, Invitrogen) à une concentration finale de 25 nM en utilisant la technique de transfection par la Lipofectamine™ RNAiMAX (Invitrogen, Ref : 13778-075) et traités ou non avec l'extrait peptidique de soja et de levure selon l'exemple 1 à une concentration finale de 1%, pendant 48 heures.
Quatre conditions ont été mises en place :
- condition 1 : contrôle sans siRNA et sans actif
- condition 2 : cellules transfectées avec le siRNA, sans actif
- condition 3 : cellules non transfectées mais traitées avec l'actif
- condition 4 : cellules transfectées avec le siRNA et traitées avec l'actif

Les cellules sont ensuite lavées, fixées au formaldéhyde à 3.7% pendant 10 minutes à température ambiante. Une perméabilisation des noyaux est réalisée à l'aide d'une incubation avec du Triton X-100 à 0,2% pendant 10 minutes à température ambiante. Les sites non spécifiques sont bloqués avec de la BSA à 1% appliquée pendant 30 minutes. Les cellules sont incubées en présence d'un anticorps monoclonal de souris spécifique de TRF2 (Abcam, réf : abl3579), puis d'un anticorps secondaire anti-souris couplé à un fluorochrome (Invitrogen, réf : A21202). Les cellules sont alors examinées au microscope à Epi-fluorescence (Nikon Eclipse E600 microscope).

Trois images par condition sont analysées et quantifiées grâce au logiciel Image-Pro Analyzer 6.3 software (MediaCybernetics, Inc.). Des analyses statistiques sont ensuite menées sur ces données en utilisant le test t de Student.

### Résultats/Conclusions :

Entre les conditions 1 et 3, on constate que l'ajout de l'extrait peptidique de soja et de levure selon l'exemple 1 a entrainé une augmentation de l'expression de TRF2 de 22 % par rapport au contrôle. Entre les conditions 1 et 2, on constate bien l'effet du siRNA spécifique FOXO3a sur le taux d'expression de la protéine TRF2 : en effet, celle-ci est en baisse de 18,9 %. Par contre, l'ajout de l'actif aux cellules transfectées avec le siRNA permet de restaurer l'expression de TRF2 (condition 4), et on observe une augmentation de 26 % par rapport aux cellules transfectées et non traitées (condition 2).

En conclusion, l'extrait peptidique de soja et de levure selon l'invention a permis de compenser la diminution de l'expression de la protéine TRF2 induite artificiellement par le siRNA spécifique de FOXO3a.

### Exemple 7 : étude de la protection de l'ADN de fibroblastes humains traités avec l'extrait peptidique de soja et de levure selon l'exemple 1

Le test comètes est un test qui permet de quantifier les dommages causés à l'ADN au niveau cellulaire.

### Protocole :

Des fibroblastes humains sont mis en culture et traités une fois par jour avec l'extrait peptidique selon l'exemple 1, à une concentration de 1 %, entre le passage 3 et le passage 16. Ils sont ensuite irradiés avec des rayonnements UVB à raison de 50 mJ/ cm², puis remis en culture dans du milieu fibroblastes pendant 30 minutes.

Une culture de contrôle est réalisée sans traitement par l'extrait peptidique selon l'exemple 1.

Les cellules sont ensuite détachées de leur support par traitement à la trypsine, afin de les concentrer et les dénombrer.

Un nombre défini de cellules (25 000 cellules) est ensuite inclus dans un gel d'agarose Low Melting à 0,75 %, puis déposé sur une lame de verre préalablement recouverte d'agarose à 1 %. Les lames sont ensuite immergées dans une solution de lyse pendant 1h30 à 4 °C, puis dans une solution alcaline pendant 20 minutes à 4 °C. Les cellules sont ainsi lysées et l'ADN dénaturé. Les lames sont plongées dans une solution d'électrophorèse avant d'appliquer un champ électrique (20 V - 250 mA). L'ADN ainsi dénaturé est soumis à une migration au sein du gel d'agarose à 4 °C. L'application d'un colorant fluorescent de l'ADN sur les lames (l'iodure de propidium à 2 µg/ ml) permet d'observer l'ADN microscope. L'ADN endommagé, c'est-à-dire fragmenté en de multiples fragments de tailles diverses, migre de façon diffuse et apparait sous forme de « comètes ».

Un logiciel de quantification permet de déterminer le Tail Moment moyen, qui est d'autant plus élevé que l'ADN est endommagé.

### Résultats :

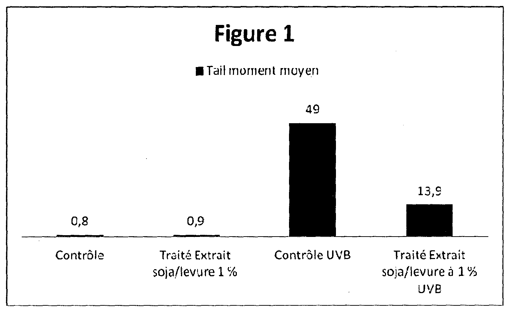

Lorsque les fibroblastes sont traités par l'extrait peptidique selon l'exemple 1 et soumis à des rayonnements UVB, le Tail Moment est de 13,9 alors qu'il est de 49 dans la condition irradiée et non traitée. Le Tail moment calculé diminue donc de 71,6 % (diminution statistiquement hautement significative) par rapport à la condition contrôle irradié, c'est-à-dire que l'ADN des cellules soumises à des rayonnements UVB a subi sensiblement moins de dommages que dans la condition contrôle, tel qu'illustré à la figure 1.

### Conclusions :

L'extrait peptidique selon l'exemple 1 à 1%, a protégé très significativement l'ADN de fibroblastes humains normaux soumis à une irradiation par les UVB.

### Exemple 8 : composition d'une crème solaire

| Noms commerciaux | Ingrédient (Noms INCI) | % massique |
|---|---|---|
| PHASE A | | |
| | Aqua (Eau déminéralisée) | qsp |
| Noms commerciaux | Ingrédient (Noms INCI) | % massique |
| | Glycerin | 3,00 |
| Versene NA2 | Disodium EDTA | 0,10 |
| | Triethanolamine | 0,50 |
| UltraThix® P100 | Acrylic acid/VP Crosspolymer | 0,70 |

| PHASE B | | |
|---|---|---|
| | Dimethicone (100cs) | 0,50 |
| Glucamate SSE20 | PEG-20 Methyl Glucose Sesquistearate | 2,50 |
| Glucamate SS | Methyl Glucose Sesquistearate | 0,50 |
| Lanette® 16 | Cetyl Alcohol | 1,50 |
| Ceraphyl® SLK | Isodecyl Neopentanoate | 3,00 |
| Ceraphyl® 230 | Diisopropyl Adipate | 2,00 |
| Escalol® 517 | Avobenzone | 3,00 |
| Escalol® 587 | Octisalate | 5,00 |
| Escalol® 597 | Octocrylene | 2,00 |
| Escalol® S | Bis-Ethylhexyloxyphenol Methoxyphenyl Triazine | 3,00 |

| PHASE C | | |
|---|---|---|
| | Cyclopentasiloxane | 5,00 |
| Si-Tec™ RE-100 | Cyclopentasiloxane, Dimethicone/Vinyltrimethylsiloxysilicate Crosspolymer | 1,00 |

| PHASE D | | |
|---|---|---|
| | Ethanol | 5,00 |
| Optiphen™ ND | Phenoxyethanol, Benzoic acid, | 1,20 |

| Noms commerciaux | Ingrédient (Noms INCI) | % massique |
|---|---|---|
| | Dehydroacetic acid | |
| PHASE E | | |
| | Extrait peptidique soja / levure selon l'exemple 1 | 1,00 |
| | Parfum (Fragrance) | qsp |
| | Colorant | qsp |

## Revendications

1. Composition cosmétique **caractérisée en ce qu'**elle comprend, dans un milieu physiologiquement acceptable, un système activateur de la protéine TRF2 à action synergique constitué par l'association d'un extrait peptidique de soja et de levure obtenu par l'hydrolyse concomitante de 90% de graine de soja (*Glycine Max L.)* en poids du poids total de matières premières et de 10% de levure du genre Saccharomyces en poids du poids total de matières premières, **caractérisée en ce que** l'extrait peptidique de soja et de levure comprend de 0,5 à 5,5 g/L de composés peptidiques dont le poids moléculaire est inférieur à 5 kDa.

2. Composition selon la revendication précédente **caractérisée en ce qu'**elle se présente sous une forme adaptée à l'application par voie topique choisie parmi une crème, émulsion huile-dans-eau, ou eau-dans-huile ou émulsion multiple, solution, suspension, microémulsion, gel aqueux ou anhydre, sérum, ou encore une dispersion de vésicules, patch, spray, onguent, pommade, lotion, colloïde, lait, stick ou poudre.

3. Utilisation cosmétique non-thérapeutique d'une composition selon l'une des revendications 1 ou 2, pour prévenir et/ou réparer la survenue des cassures doubles brins au niveau de l'ADN des cellules cutanées.

4. Utilisation cosmétique non-thérapeutique selon la revendication 3, pour prévenir et/ou traiter les signes cutanés du vieillissement et du photo-vieillissement induits par les rayonnements UV.

5. Utilisation cosmétique non-thérapeutique selon l'une des revendications 3 ou 4, pour prévenir et/ou traiter les rides, les rides profondes, les ridules, le relâchement des tissus cutanés et sous-cutanés, la perte de l'élasticité cutanée et l'atonie, la perte de fermeté et de tonicité, et l'atrophie dermique.

6. Méthode de soin cosmétique non-thérapeutique pour prévenir et/ou réparer les dommages subis par l'ADN des cellules cutanées liés au vieillissement comprenant l'application topique sur au moins une partie de la peau du visage ou du corps de la composition selon la revendication 1 ou 2, dans laquelle la composition est appliquée le matin en tant que soin anti-âge de jour et/ou le soir au coucher en tant que soin réparateur de nuit.

7. Méthode de soin cosmétique non-thérapeutique pour prévenir et/ou réparer les dommages subis par l'ADN des cellules cutanées liés au vieillissement comprenant l'application topique sur au moins une partie de la peau du visage ou du corps de la composition selon la revendication 1 ou 2, dans laquelle la composition est appliquée avant une exposition au soleil, en tant que soin avant-soleil et/ou après une exposition au soleil, en tant que soin après-soleil.

## Patentansprüche

1. Kosmetische Zusammensetzung, **dadurch gekennzeichnet, dass** diese, in einem physiologisch annehmbaren Medium, ein Aktivatorsystem des Proteins TRF2 mit synergistischer Wirkung umfasst, das hergestellt wird durch die Assoziation eines Peptidextrakts von Soja und Hefe, der durch gleichzeitige Hydrolyse von 90 Gew.-% Sojabohnen (Glycine Max L.), bezogen auf das Gesamtgewicht der Ausgangsstoffe, und 10 Gew.-% Hefe der Gattung Saccharomyces, bezogen auf das Gesamtgewicht der Ausgangsstoffe, erhalten wird, **dadurch gekennzeichnet, dass** der Peptidextrakt von Soja und Hefe 0,5 bis 5,5 g/l Peptidverbindungen umfasst, deren Molekulargewicht kleiner ist als 5 kDa.

2. Zusammensetzung nach dem vorhergehenden Anspruch, **dadurch gekennzeichnet, dass** diese eine Form aufweist, die für eine Verabreichung auf topischem Weg geeignet ist, ausgewählt aus einer Creme, einer Öl-in-Wasser- oder Wasser-in-Öl-Emulsion oder Mehrfachemulsion, einer Lösung, einer Suspension, einer Mikroemulsion, einem wässrigen oder wasserfreien Gel, einem Serum oder auch einer Vesikeldispersion, einem Pflaster, einem Spray, einer Salbe, einer Pommade, einer Lotion, einem Kolloid, einem Stift oder einem Puder.

3. Nicht-therapeutische kosmetische Verwendung einer Zusammensetzung nach einem der Ansprüche 1 oder 2, zur Verhinderung und/oder Reparatur des Auftretens von Doppelstrangbrüchen auf der Ebene der DNS von Hautzellen.

4. Nicht-therapeutische kosmetische Verwendung nach Anspruch 3, zur Verhinderung und/oder Behandlung von Anzeichen einer Hautalterung und Lichtalterung, die durch UV-Strahlen induziert werden.

5. Nicht-therapeutische kosmetische Verwendung nach einem der Ansprüche 3 oder 4, zur Verhinderung und/oder Behandlung von Falten, tiefen Falten, Fältchen, der Lockerung des kutanen und subkutanen Gewebes, des Verlusts der Hautelastizität und Atonie, des Verlusts der Festigkeit und Tonizität und der Hautatrophie.

6. Verfahren zur nicht-therapeutischen kosmetischen Pflege zur Verhinderung und/oder Reparatur von Schäden, welche die DNS von Hautzellen erlitten hat und mit der Alterung verbunden sind, umfassend das topische Aufbringen, auf mindestens einen Teil der Haut des Gesichts oder des Körpers, der Zusammensetzung nach Anspruch 1 oder 2, wobei die Zusammensetzung am Morgen als Anti-Alterungs-Tagespflege und/oder am Abend vor dem Schlafengehen als Reparatur-Nachtpflege angewendet wird.

7. Verfahren zur nicht-therapeutischen kosmetischen Pflege zur Verhinderung und/oder Reparatur von Schäden, welche die DNS von Hautzellen erlitten hat und mit der Alterung verbunden sind, umfassend das topische Aufbringen, auf mindestens einen Teil der Haut des Gesichts oder des Körpers, der Zusammensetzung nach Anspruch 1 oder 2, wobei die Zusammensetzung vor dem Aussetzen an die Sonne, als Sonnenschutzpflege, und/oder nach einem Aussetzen an die Sonne, als Aftersun-Pflege, angewendet wird.

## Claims

1. A cosmetic composition **characterized in that** it comprises, in a physiologically acceptable medium, a synergistic activator system of the TRF2 protein consisting of the combination of soy and yeast peptide extract obtained by simultaneous hydrolysis of 90 % by weight of the total weight of raw materials of soy seed (Glycine Max L.) and 10 % by weight of the total weight of raw materials of yeast of the Saccharomyces genus, **characterized in that** the soy and yeast peptide extract comprises 0.5 to 5.5 g/L peptide compounds with a molecular weight of less than 5 kDa.

2. The composition according to the preceding claim, **characterized in that** it is in a form suitable for a topical application chosen among a cream, oil-in-water or water-in-oil or multiple emulsion, suspension, micro-emulsion, aqueous or anhydrous gel, serum or also a dispersion of vesicles, patch, spray, ointment, salve, lotion, colloid, milk, stick or powder.

3. A non-therapeutic cosmetic use of a composition according to one of claims 1 or 2 for preventing and/or repairing the occurrence of double-strand breaks in the DNA of skin cells.

4. The non-therapeutic cosmetic use according to claim 3 for preventing and/or treating cutaneous signs of ageing and photo-ageing caused by UV radiation.

5. The non-therapeutic cosmetic use according to one of claims 3 or 4 for preventing and/or treating wrinkles, deep wrinkles, fine lines, the slackening of cutaneous and subcutaneous tissue, loss of skin elasticity and atony, loss of firmness and tone and dermal atrophy.

6. A method of non-therapeutic cosmetic care for preventing and/or repairing damage to the DNA of skin cells associated with ageing comprising topical application to at least a portion of the skin of the face or body of the composition according to claim 1 or 2, in which the composition is applied in the morning as a daytime anti-ageing treatment and/or at bedtime as a night-time repair treatment.

7. The method of non-therapeutic cosmetic care for preventing/repairing damage to the DNA of skin cells associated with ageing comprising the topical application to at least a portion of the skin of the face or body of the composition according to claim 1 or 2, wherein the composition is applied prior to exposure to the sun as a pre-sun treatment and/or following exposure to the sun as an after-sun treatment.
